# FASCICULE DE BREVET EUROPEEN

(11) **EP 3 596 197 B1**
(45) Date de publication et mention de la délivrance du brevet: **27.10.2021**
(21) Numéro de dépôt: 18712976.2
(22) Date de dépôt: 08.03.2018
(51) Int. Cl.: C12M 3/00, A61F 2/50, B33Y 80/00

(54) **PROCEDE DE BIOIMPRESSION**
BIOPRINTING-VERFAHREN
BIOPRINTING PROCESS

(30) Priorité: 15.03.2017 FR 1752130
(43) Date de publication de la demande: 22.01.2020
(73) Titulaire: Poietis, 33600 Pessac (FR)
(72) Inventeur: GUILLEMOT, Fabien, 33210 Preignac (FR); VIELLEROBE, Bertrand, 33700 Merignac (FR); SIMON, Guillaume, 33700 Merignac (FR); VANDENEECKHOUTTE, Guillaume, 33700 Merignac (FR)
(74) Mandataire: IP Trust
(86) Numéro de dépôt international: PCT/FR2018/050534
(87) Numéro de publication internationale: WO 2018/167401

(56) Documents cités:
- WO-A1-2011/107599
- WO-A1-2013/158508
- WO-A2-2005/081970
- WO-A2-2015/148646

## Description

### Domaine de l'invention

La présente invention concerne le domaine de la bioimpression par laser par un procédé de transfert assisté par ordinateur pour la modélisation et l'assemblage de matériaux vivants et optionnellement non-vivants avec une organisation prescrite 2D ou 3D dans le but de produire des structures de bioingénierie servant en médecine régénérative, en pharmacologie et pour des études de biologie cellulaire comme décrit dans le document WO-A-2005/081970.

L'ingénierie tissulaire vise à concevoir et développer des solutions de substitution biologiquement aptes à remplacer, restaurer ou maintenir les fonctions d'un tissu natif, voire d'un organe. Un exemple est décrit dans l'article Griffith, L. G., & Naughton, G. (2002). Tissue engineering--current challenges and expanding opportunities. Science, 295(5557), 1009-1014.

Pour qu'elles soient efficaces, ces méthodes artificielles de (re)construction se doivent de reproduire le plus fidèlement possible la complexité propre au tissu ciblé, caractérisée et résultant des boucles d'action et de contre-réaction exercées par les différents constituants du tissu (cellules, matrice extracellulaire, morphogènes) de l'échelle microscopique à l'échelle macroscopique. Ces interactions sous-entendent une relation étroite entre la forme ou l'organisation des tissus et leur fonction. Le fait est que les approches traditionnelles d'ingénierie tissulaire présentent un certain nombre de limitations, en particulier la difficulté de contrôler la complexité des tissus, leur vascularisation, la personnalisation qui en découle ainsi que la sécurité dans leur production.

Pour pallier ces limites, l'impression d'éléments biologiques, plus communément appelée bioimpression, a commencé à être imaginée, comme exposé dans les articles Klebe, R. (1988). Cytoscribing: A Method for Micropositioning Cells and the Construction of Two- and Three-Dimensional Synthetic Tissues. Expérimental Cell Research, 179(2):362-373.

Et Klebe, R., Thomas, C., Grant, G., Grant, A. et Gosh, P. (1994). Cytoscription: Computer controlled micropositioning of cell adhesion proteins and cells. Methods in Cell Science, 16(3):189-192.

Cette technique permet d'organiser les différents constituants d'un tissu biologique (cellules vivantes, biomatériaux, molécules) tout en garantissant le maintien de leurs propriétés post-impression, puis d'assembler en 3D, couche par couche, ces différents constituants du tissu. Les avantages majeurs de la bioimpression sur les méthodes de bio-fabrication plus classiques sont la reproductibilité (automatisation), la rapidité d'exécution (planification informatique / parallélisation) et la capacité de personnalisation.

On dénombre aujourd'hui trois technologies phares de bioimpression : le jet d'encre, la bio-extrusion et l'impression assistée par laser (LAB) comme exposé dans l'article Dababneh, A. B., & Ozbolat, I. T. (2014). Bioprinting technology: a current state-of-the-art review. Journal of Manufacturing Science and Engineering, 136(6).

### Etat de la technique

On connaît dans l'état de la technique une solution désignée par le nom de « AFA-LIFT » et décrite dans l'article B. Hopp, T. Smausz, N. Kresz, N. Barna, Z. Bor, L. Kolozsvári, D. B. Chrisey, A. Szabó, and A. Nógrádi, "Survival and proliferative ability of various living cell types after laser-induced forward transfer," Tissue Eng. 11, 1817-23 (2005).

L'article « J. A. Barron, P. Wu, H. D. Ladouceur, and B. R. Ringeisen, "Biological laser printing: a novel technique for creating heterogeneous 3-dimensional cell patterns," Biomed. Microdevices 6, 139-147 (2004) » décrit également un équipement de type « AFA LIFT ou DRL-LIFT », où la direction du laser est fixe, et le film supportant les cellules à transférer est mobile.

Un autre article, publié dans le « Journal of laser micro/nanoengineering » Vol 9, N°2-2014 sous le titre « Laser tool for single cell transfer » décrit un autre exemple de mise en œuvre de procédé et d'équipement de type AFA-LIFT.

L'article « F. Guillemot, A. Souquet, S. Catros, B. Guillotin, J. Lopez, M. Faucon, B. Pippenger, R. Bareille, M. Rémy, S. Bellance, P. Chabassier, J. C. Fricain, and J. Amédée, "High- throughput laser printing of cells and biomaterials for tissue engineering," Acta Biomater. 6, 2494-2500 (2010) » décrit un exemple d'équipement pour mettre en œuvre un tel procédé.

On connaît aussi dans l'état de la technique la demande de brevet WO2016097619 décrivant un procédé et un équipement d'impression d'au moins une encre, ledit procédé comprenant une étape de focalisation d'un faisceau laser de manière à générer une bulle de cavitation dans un film d'encre, une étape de formation d'au moins une gouttelette d'encre à partir d'une surface libre du film d'encre et une étape de dépôt de ladite gouttelette sur une surface de dépose d'un substrat receveur, caractérisé en ce que le faisceau laser est orienté à contresens par rapport à la force gravitationnelle, la surface libre du film étant orientée vers le haut en direction de la surface de dépose placée au-dessus du film d'encre.

Cette configuration permet notamment d'obtenir une épaisseur E pour le film d'encre sensiblement constante, tout en limitant l'apparition des phénomènes de sédimentation. De plus, elle permet d'utiliser une large gamme d'encres.

### Inconvénients de l'art antérieur

Les solutions de l'art antérieur ne sont pas bien adaptées à la fabrication de tissus à partir de cellules vivantes, qui évoluent après l'étape de bioimpression. Les cellules vivantes déposées par transfert peuvent adhérer, proliférer, se déformer, se déplacer, se différencier, rentrer en quiescience et en apoptose. De ce fait, l'objet biologique produit par bioimpression n'est pas figé, et le tissu obtenu après une phase de maturation ne correspond pas nécessairement à celui qui a été réalisé.

Les solutions de l'art antérieur ne permettent pas de construire un modèle prédéterminé lorsque les constituants du tissu évoluent dans le temps (impression 4D) sous l'action de ses mêmes constituants ou sous l'action de stimuli externes (exogènes) comme la température, le pH, le taux d'oxygène ou de CO2...

### Solution apportée par l'invention

La présente invention concerne selon son acception la plus générale un procédé de bioimpression comportant :
- une étape de préparation d'un modèle numérique cible représentatif de l'organisation tridimensionnelle du tissu à fabriquer, (appelé aussi fichier CAO),
- une étape de pilotage d'un équipement de bioimpression pour le dépôt d'une pluralité de couches de cellules vivantes et de biomatériaux
- une étape optionnelle de caractérisation 2D de chacune des couches pendant l'étape de bioimpression
- caractérisé en ce qu'il comporte en outre
- une étape de calcul d'un modèle numérique d'impression en fonction dudit modèle numérique du produit à fabriquer, d'une part, et d'un modèle prédictif d'évolution d'autre part, ainsi que des caractéristiques des constituants à imprimer (intérêt pour la personnalisation)
   ∘ l'étape de pilotage de l'équipement de bioimpression étant réalisée avec ledit modèle numérique d'impression ainsi calculé.

Une étape de maturation du produit d'impression ainsi fabriqué peut également être prévue à l'issue de l'impression lorsqu'on prépare un modèle in vitro. Pour l'impression in situ (in vivo) il n'y a pas d'étape de maturation. Quant à l'impression de modèles in vitro pour une implantation directe, l'étape de maturation est partielle.

Le procédé peut optionnellement comporter une étape de représentation et d'enregistrement de la suite de tâches et opérations et des données correspondantes, notamment les tâches de préparation de la banque d'objets biologiques, les tâches de CAO, d'impression et de maturation) qui permet de tracer l'histoire de chacune des impressions.

De préférence, le procédé de bioimpression comporte en outre une étape de caractérisation 3D du produit bioimprimé au cours de sa maturation pour les modèles in-vitro.

Les étapes de caractérisation 2D et 3D peuvent recouvrir plusieurs types de formes comme l'utilisation de moyens d'analyse topographiques, tomographiques, spectroscopiques (Raman, Rayleigh, IR...), d'analyse bio-chimique... Elles seront préférentiellement basées sur des techniques d'imagerie 2D et 3D permettant d'analyser le tissu en cours d'impression et à l'issue de l'impression. Ces techniques peuvent être basées sur des techniques d'imagerie optique (confocale, fluorescence, OCT, Imagerie de Phase, SPIM, Multiphoton...), acoustique, par rayons X, etc...

Selon une variante ledit modèle prédictif d'évolution est enregistré sur un serveur partagé, lesdits équipements comportant des moyens de communication avec ledit serveur pour sélectionner dans une bibliothèque numérique un modèle prédictif d'évolution enregistré adapté à une catégorie de produits cibles.

Selon une autre variante ledit modèle prédictif d'évolution détermine décalage entre la position de transfert de biomatériau théorique et la position réelle dudit biomatériau sur la cible réceptrice observée par la caméra visualisant la cible.

Selon d'autres variantes, le procédé comporte :
- des étapes de transmission des caractérisations 2D et 3D acquises sur un équipement à un serveur partagé entre un pluralité de serveurs.
- des étapes de traitement de type multi-agents sur les données provenant d'au moins un équipement connecté pour le recalcul périodique des modèles prédictif d'évolution.
- des étapes de traitement de type apprentissage par machine sur les données provenant d'au moins un équipement connecté pour le recalcul périodique des modèles prédictif d'évolution.

Ce recalcul périodique réalise un apprentissage continu avec un calcul en permanence sur les données déjà acquises par assimilation des données. Ainsi le modèle prédictif utilise les apprentissages précédents pour mieux prédire.
- des étapes de traitement de type modélisation continue par équations différentielles sur les données provenant d'au moins un équipement connecté pour le recalcul périodique des modèles prédictif d'évolution.
- des étapes de traitement de l'espace d'imagerie pour déterminer des bornes des tissus viables et non viables afin de recalculer périodiquement l'espace d'impression optimal en fonction desdites bornes des tissus viables.

Avantageusement, les équipements connectés transmettent audit serveur connecté :
- des informations comprenant le modèle numérique d'un produit à fabriquer, ainsi que les caractérisations 2D d'impression et 3D post impression et en phase de maturation lorsqu'il y en a une.
- des informations sur les conditions physiques de l'équipement (pendant les étapes de bioimpression et de maturation lorsqu'il y en a une).
- des informations issues du cahier de laboratoire électronique (ELN) sur la préparation de l'expérience, la préparation des cellules, des biomatériaux et des milieux de culture
- des informations numériques horodatées, descriptives du tissu cible et des événements détectés pendant l'étape de bio-impression et l'étape de maturation lorsqu'il y en a une.

Selon une variante, le procédé selon l'invention comporte une étape de calcul d'une pluralité de modèles d'impression et des étapes d'évaluation des évolutions théoriques des modèles d'impression ainsi calculés, et de sélection du modèle d'impression (17) minimisant les écarts entre le modèle cible et le modèle maturé calculé.

Selon une variante, l'étape de calcul du modèle d'impression met en œuvre un traitement de convolution entre le modèle cible et un modèle prédictif d'évolution tout en tenant compte des intrants liés à la future impression (constituants réels, machine, procédés...) afin de garantir un calcul optimal dudit modèle d'impression.

L'invention se rapporte aussi à un système de bioimpression comportant :
- un poste de bioimpression équipé
   ∘ d'au moins une tête de bioimpression apte à commander le transfert de biomatériaux et/ou de cellules vivantes depuis un substrat vers une cible,
   ∘ et de moyens de caractérisation 2D pour l'enregistrement de données (morphologiques, moléculaires ou chimiques) de chacune des couches pendant l'étape de bioimpression
- au moins un poste de maturation des cibles réalisées par bioimpression si les dites cibles sont destinées à des tests in vitro (sans implantation directe ou indirecte)
- ainsi qu'un calculateur commandant ledit poste de bioimpression, comportant des moyens de définition et d'enregistrement dans la mémoire du calculateur d'un modèle numérique cible représentatif de l'organisation tridimensionnelle du produit à fabriquer,
   caractérisé en ce qu'il comporte un calculateur associé à une mémoire pour l'enregistrement d'une pluralité de modèles numériques prédictif d'évolution et exécute un programme commandant
- une étape de calcul d'un modèle numérique d'impression calculé en fonction
   ∘ dudit modèle numérique cible du produit à fabriquer, d'une part, et
   ∘ d'un modèle prédictif d'évolution d'autre part, et
   ∘ des caractéristiques des constituants imprimés, et
      - une étape de pilotage dudit poste de bioimpression en application dudit modèle numérique d'impression ainsi calculé.

Avantageusement, le système comporte :
- une pluralité de postes de bioimpression, chacun desdits postes de bioimpression comportant des moyens de communication avec un serveur commun comportant ledit calculateur, ledit calculateur transmettant à chaque calculateur local associé à un poste de bioimpression le modèle numérique à appliquer par la tête de bioimpression en fonction de chaque application visée.
- une pluralité de postes de bioimpression munis de moyens de transmission des caractérisations 2D acquises en cours de bioimpression audit serveur, ainsi que des capteurs physiques pour la transmission audit serveur des informations horodatées du contexte de bioimpression.
- une pluralité de postes de maturation comportant des moyens de transmission des images 3D acquises en cours de maturation audit serveur, ainsi que des capteurs physiques pour la transmission audit serveur des informations horodatées du contexte de maturation lorsqu'il y en a un. Des capteurs physiques peuvent aussi être intégrés directement au coeur de l'objet imprimé et peuvent participer à la collecte de données décrite ici.

Selon une variante, le procédé comporte des étapes de traitement de type modélisation continue par équations différentielles sur les données provenant d'au moins un équipement connecté pour le recalcul périodique des modèles prédictif d'évolution.

Selon une autre variante, le procédé comporte des étapes de traitement de type modélisation de type multi-agents sur les données provenant d'au moins un équipement connecté pour le recalcul périodique des modèles prédictif d'évolution.

Ces modèles prédictifs d'évolution sont des modèles spécifiques au type cellulaires, soit par modèles multi-agents soit par modèles continus.

Avantageusement, le procédé comporte le traitement selon une pluralité de modèles, par exemple d'un ou plusieurs modèles multi-agents et d'un ou plusieurs modèles continus et de prendre la prédiction pondérée par la véracité des modèles selon l'espace de leurs paramètres viables.

Selon une autre variante, le procédé comporte des étapes de traitement de type modélisation de type apprentissage par machine (machine learning) sur les données provenant d'au moins un équipement connecté pour le recalcul périodique des modèles prédictif d'évolution.

Selon une variante, lesdits modèles sont des modèles intégrés multi-échelles, par exemple, l'échelle génétique (spécification cellulaire par GRN), peut permettre de définir un espace de paramètres pour l'échelle cellulaire (Multi-agents) qui peuvent eux être les points d'entrée d'un modèle continu (tissulaire)

Avantageusement, les équipements connectés transmettent audit serveur connecté des informations comprenant le modèle numérique d'un produit à fabriquer, ainsi que les caractérisations 2D d'impression et les caractérisations 3D post-impression et en maturation.

Selon une variante, les équipements connectés transmettent audit serveur connecté des informations sur les conditions physiques de l'équipement pendant les étapes de bioimpression et de maturation.

Avantageusement, les équipements connectés transmettent audit serveur connecté des informations numériques horodatées, descriptives du tissu cible, de son environnement (milieu, conditions environnementales) et des événements détectés pendant l'étape de bio-impression et l'étape de maturation.

Selon un mode de réalisation particulier, le procédé comporte une étape de calcul d'une pluralité de modèles d'impression et des étapes d'évaluation des évolutions théoriques des modèles d'impression ainsi calculés, et de sélection du modèle d'impression minimisant les écarts entre le modèle cible et le modèle maturé calculé.

Selon une variante, l'étape de calcul du modèle d'impression met en œuvre un traitement de convolution entre le modèle cible et un modèle prédictif d'évolution.

L'invention concerne aussi un système de bioimpression comportant :
- un poste de bioimpression équipé
   ∘ d'au moins une tête de bioimpression apte à commander le transfert de biomatériaux et/ou de cellules vivantes depuis un substrat vers une cible,
   ∘ et de moyens de caractérisation 2D pour l'enregistrement de données (morphologiques, moléculaires ou chimiques) de chacune des couches pendant l'étape de bioimpression
- au moins un poste de maturation des objets cibles réalisés par bio-impression si les dites cibles sont destinées à des tests in vitro (sans implantation directe ou indirecte)
- ainsi qu'un calculateur commandant ledit poste de bioimpression, comportant des moyens de définition et d'enregistrement dans la mémoire du calculateur d'un modèle numérique cible représentatif de l'organisation tridimensionnelle du produit à fabriquer,
caractérisé en ce que ledit calculateur comporte une mémoire pour l'enregistrement d'une pluralité de modèles numériques prédictifs d'évolution et exécute un programme commandant :
- une étape de calcul d'un modèle numérique d'impression calculé en fonction
   ∘ dudit modèle numérique cible du produit à fabriquer, d'une part, et
   ∘ d'un modèle prédictif d'évolution d'autre part, et
   ∘ des caractéristiques des constituants imprimés, et
      - une étape de pilotage dudit poste de bioimpression en application dudit modèle numérique d'impression ainsi calculé.

Avantageusement, le système selon l'invention comporte une pluralité de postes de bioimpression, chacun desdits postes de bioimpression comportant des moyens de communication avec un serveur commun comportant ledit calculateur, ledit calculateur transmettant à un second calculateur local associé à un poste de bioimpression le modèle numérique à appliquer par la tête de bioimpression.

Avantageusement, il comporte une pluralité de postes de bioimpression munis de moyens de transmission des caractérisations 2D acquises en cours de bioimpression audit serveur, ainsi que des capteurs physiques pour la transmission audit serveur des informations horodatées du contexte de bioimpression. Des capteurs physiques peuvent aussi être intégrés directement au coeur de l'objet imprimé et peuvent participer à la collecte de données décrite ici.

Selon une variante, le système selon l'invention comporte une pluralité de postes de maturation comportant des moyens de transmission des caractérisations 3D acquises post impression et en cours de maturation lorsqu'il y en a audit serveur, ainsi que des capteurs physiques pour la transmission audit serveur des informations horodatées du contexte de maturation.

### Description détaillée d'un exemple non limitatif de réalisation

D'autres caractéristiques et avantages ressortiront de la description qui va suivre de l'invention, description donnée à titre d'exemple uniquement, se référant aux dessins annexés sur lesquels :

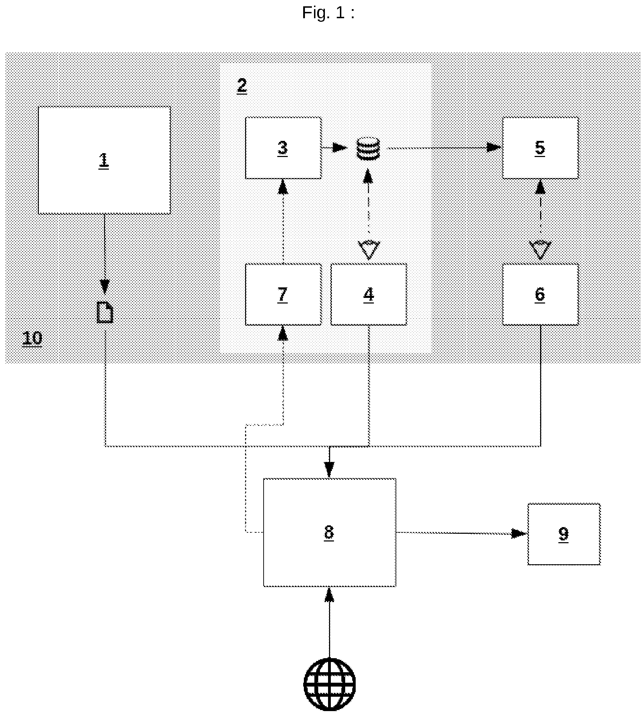

- La figure 1 représente une vue schématique d'un système selon l'invention
- La figure 2 représente une vue schématique d'un système selon une variante associant une pluralité d'équipements à un serveur partagé
- La figure 3 représente une vue schématique d'un procédé de bioimpression selon l'invention
- la figure 4 représente un schéma fonctionnel illustrant les différentes séquences du procédé selon l'invention.

### Contexte de l'invention

La précision apportée par l'impression assistée par laser, permettant de positionner des cellules uniques, ouvre un nouveau champ de recherches très large sur l'organisation cellulaire au sein d'un tissu. Grâce à des techniques de caractérisations multiples, notamment de cellules vivantes, une base de données massive sur ce sujet se constitue au cours du temps. Pour être exploitées, ces données brutes peuvent être organisées en modèles, c'est à dire en constructions abstraites capables d'articuler les données les unes par rapport aux autres. Ainsi il devient possible d'identifier des comportements dans le temps et dans l'espace, de donner une explication (biologique, mécanique, physique, etc.) sur tel ou tel processus d'organisation ou de faire des prévisions sur le système réel. De plus, certains de ces modèles peuvent se traiter de manière mathématique ou informatique permettant ainsi d'utiliser la capacité de résolution algébrique des mathématiques ou la puissance de calcul des ordinateurs comme exposé dans l'article « Ballet, P., Pothet, A., Misevic, G., Jeannin-Girardon, A., Fronville, A., & Rodin, V. (2013). Une approche multi-agent pour la simulation en biologie cellulaire. Le vivant discret et continu, 155-194 ».

### Description de l'équipement selon l'invention

L'invention est mise en œuvre sur un système comprenant principalement les postes suivants :
- un poste informatique (1) pour la définition d'un modèle cible correspondant à la configuration d'un tissu vivant attendu après la maturation ou l'implantation du modèle obtenu par bioimpression
- un poste de bioimpression (2) comportant :
   - une ou plusieurs têtes de bioimpression (3) permettant de réaliser le transfert de biomatériaux ou de cellules vivantes depuis un substrat vers une cible
   - une caméra (4) observant la cible pour réaliser des images 2D pendant la phase de bioimpression, notamment pour la vérification des transferts couche par couche, la caractérisation 2D pouvant revêtir de nombreuses autres formes que l'imagerie
   - un calculateur local (7) pilotant la tête d'impression (3)
- un poste de maturation (5) recevant la cible à l'issue de la phase de bioimpression et fixant des paramètres de maturation du tissu ainsi réalisé. Ce poste de maturation (5) comprend également un système d'imagerie 3D (6) pour l'acquisition périodique d'une image 3D du tissu en cours de maturation. La caractérisation peut être réalisée avec des solutions autres que l'imagerie. Par ailleurs cette étape de maturation n'a lieu que lorsque le tissu est à destination de tests in vitro.

Les moyens techniques susvisés constituent un premier sous-ensemble (10), local. Dans la présente description, le poste de bioimpression présenté met en œuvre une technologie de transfert assisté par laser, mais cet exemple de technologie n'est pas limitatif à l'invention.
- un calculateur central (8) recevant les données provenant du poste informatique (1), de la caméra (4) du poste de bioimpression, du système d'imagerie 3D (6) du poste de maturation et transmettant au calculateur local (7) les fichiers numériques de pilotage de la tête de bioimpression (3). Bien entendu, le calculateur local (7) et le calculateur central (8) peuvent être confondus en un seul équipement. Le calculateur central (8) peut également être distant, sous forme d'un serveur, et notamment pour le partage de cette ressource avec une pluralité de postes de bioimpression et de maturation.

Le calculateur central (8) est associé à une mémoire (9) pour l'enregistrement de modèles numériques d'évolution.

Le calculateur (8) reçoit également des données provenant de sources internes par exemple les données biologiques du cahier de laboratoire sur l'origine des biomatériaux ou cellules à transférer ternes, ou provenant de sources externes par exemple des données bibliographiques ou des fichiers correspondant à des modèles d'évolution développés par des tiers. Les données biologiques sont couramment désignées par le néologisme « omes » ou « omics » et concernent par exemple les données génomiques, protéiniques, ou métaboliques ou encore les données transcriptomiques (issues du séquençage ARN).

Le calculateur (8) est associé à un poste de gestion des données (base de données par exemple) avec une structure du stockage.

Le poste de bioimpression est par exemple de type AFA-LIFT (nom commercial) ou un équipement décrit dans la demande de brevet WO2016097619, permettant de transférer des particules ou cellules de manière unitaire, mais l'invention n'est pas limitée à une technologie particulière de bioimpression.

### Variante connectée

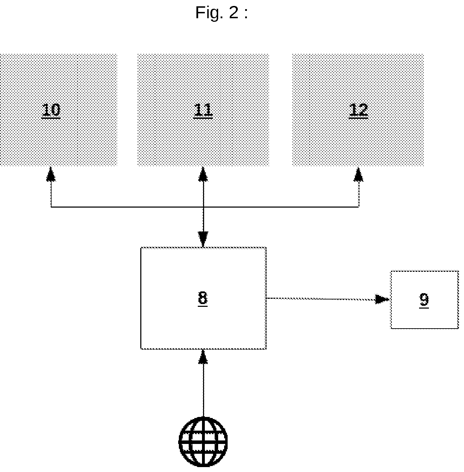

La figure 2 représente une vue schématique d'une variante de réalisation où plusieurs équipements (10 à 12) sont connectés à un serveur commun (8), réalisant des traitements sur les données provenant des différents équipements connectés pour l'élaboration de modèles numériques d'évolution calculés en fonction des données provenant de l'ensemble des équipements (10 à 12), et pas seulement d'un équipement spécifique.

### Procédé mis en œuvre par l'impression

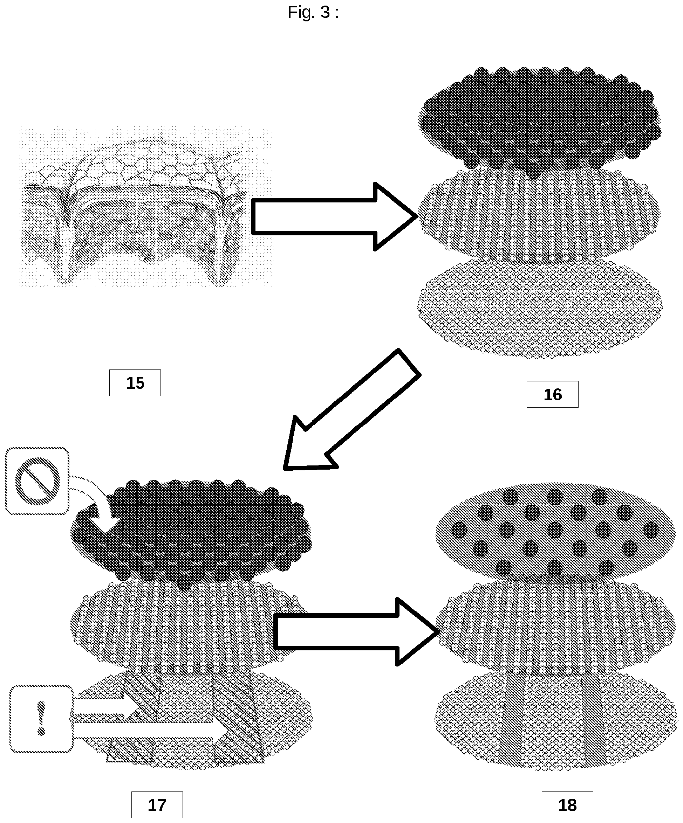

La figure 3 représente une vue schématique des traitements mise en œuvre par l'invention.

La première étape consiste à déterminer un type de tissu attendu, par exemple sous la forme d'un métamodèle (15) représentatif de l'objet final attendu. Ce métamodèle détermine notamment la localisation tridimensionnelle des biomatériaux ou cellules vivantes dans l'objet cible et tient compte de la nature des dits constituants, notamment leur niveau différentiation cellulaire dans l'objet final.

Par exemple, si le tissu attendu est une peau, le métamodèle déterminera la composition des couches constitutives comprenant :
- une couche de fibroblastes (cellules) et de collagène et de protéoglycanes (biomatériaux) constituant la matrice extracellulaire, cette couche correspondant au derme,
- une couche de kératinocytes (cellules) correspondant à l'épiderme,
- une zone intermédiaire correspondant à la jonction dermo-épidermique.

Le premier traitement consiste à construire une représentation numérique de ce métamodèle, constituant le modèle numérique cible (16). Ce modèle numérique cible se traduit par exemple par une matrice numérique de type [IDᵢ, Cᵢ, Xᵢ, Yᵢ, Zᵢ] où :
- IDᵢ correspond à un identifiant de chaque objet,
- Cᵢ correspond aux caractéristiques de l'objet IDᵢ (par exemple la nature de l'objet, sa maturité, sa dimension, etc)
- Xᵢ, Yᵢ, Zᵢ correspondent aux coordonnées spatiales de l'objet dans la cible.

Ce modèle numérique cible (16) peut être élaboré objet par objet par l'utilisateur, ou par un outil informatique générant ce modèle numérique cible (16) à partir d'une collection de métamodèles dont le modèle numérique cible paramétrable est précalculé et enregistré dans une mémoire informatique.

L'utilisateur peut ainsi modifier certaines données du modèle numérique précalculé, par exemple des caractéristiques spécifiques de certains objets, ou une modification de la densité d'objets ou la taille globale du modèle (ajout de matière, retrait de matière, sélection de couches à ne pas imprimer...) ou encore par la fusion ou l'association de plusieurs modèles précalculés.

Dans l'état de la technique, ce modèle numérique cible (16) est utilisé pour le pilotage de la tête de bioimpression. Cette solution ne permet généralement pas de fabriquer un tissu conforme à la configuration attendue. En effet, les cellules et biomatériaux transférés selon l'implantation déterminée par ce modèle numérique (16) évoluent, et le tissu se transforme entre l'étape de bioimpression et la fin de la maturation ou de l'implantation du tissu bioimprimé.

L'objet de l'invention est de remédier à ce problème, par une solution consistant à ne pas utiliser ce modèle numérique cible (16) pour piloter le poste de bioimpression, mais à calculer un modèle numérique d'impression (17) par un traitement numérique appliqué sur le modèle numérique cible (16).

Ce traitement consiste en une transformation matricielle, prenant en compte :
a) le modèle numérique cible (16) susvisé
b) un modèle numérique d'évolution (18) des éléments figurant dans le modèle numérique cible (16)
c) les caractéristiques des constituants à imprimer.

### Modèle numérique d'évolution

Le modèle numérique d'évolution comprend :
- des représentations numériques du comportement endogène de chacun des biomatériaux ou cellules vivantes susceptibles d'être transférés, enregistrés sous forme de fichiers numériques dans la base de données (9) ou dans des fichiers de logs. Ces représentations numériques sont constituées par exemple par une fonction définissant l'évolution temporelle d'une caractéristique C d'un élément donné (taille, mortalité, différentiation, division,...) ou l'évolution spatiale due à un déplacement, une migration ou une prolifération, ou encore la création automatique de nouveaux éléments dérivés de l'élément transféré
- optionnellement des représentations numériques du comportement exogène d'un élément en fonction de son environnement (présence dans un rayon donné d'un ou plusieurs éléments du même type, présence dans un rayon donné d'un ou plusieurs éléments d'un type différent, conditions physico-chimique observées pendant la maturation,...)

Ces représentations numériques peuvent être de type déterministe, par la connaissance générique du comportement biologique d'un ou plusieurs éléments, ou empirique, par une observation des comportements et par apprentissage.

Dans les deux cas, la représentation numérique se traduira techniquement par une matrice numérique permettant des traitements numériques pour la transformation d'un modèle numérique cible (16) en un modèle numérique d'impression (17).

Le traitement numérique consiste par exemple en un traitement de type transformation matricielle ou encore des traitements de type multi-agents itératif, ou encore des traitements de type « jeu de la vie » (automate cellulaire).

A titre d'exemple, le brevet US7000093 et le brevet US5432718 décrivent un automate cellulaire adapté à l'élaboration de modèles numériques d'évolution.

Ce modèle (17) transformé constitue le modèle pilotant le poste de bioimpression.

### Bioimpression et maturation

L'étape de bioimpression est réalisée avec un équipement assurant le transfert de biomatériau(x) et/ou de cellules vivantes en fonction d'une séquence définie à partir du modèle numérique d'impression (17) et d'une optimisation de l'enchaînement temporel des tirs de manière à minimiser le temps d'impression afin d'assurer une viabilité cellulaire maximale ce qui revient à minimiser les déplacements relatifs de la tête d'impression par rapport au substrat contenant les éléments transférables et/ou optimiser les déplacements en fonction de l'évolution de la cible.

Le poste d'impression comporte une caméra (4) d'observation de la cible, procédant à l'acquisition périodique de l'image 2D de la cible. Cette image est transmise à l'ordinateur local (7) pour l'évaluation de la conformité du transfert par rapport au modèle numérique d'impression (17) et éventuellement recalculer la séquence de tirs laser futurs pour corriger les anomalies observées. La caractérisation 2D peut revêtir d'autres formes que l'imagerie comme l'analyse biochimique, l'analyse spectroscopique, etc. La correction de l'impression peut aussi passer par une aspiration des zones où il y aurait eu trop d'éléments imprimés.

Ces images 2D sont aussi transmises au calculateur (8) pour l'enregistrement en relation avec les informations relatives à la bioimpression en cours. Ces informations enregistrées comprennent :
- Les données provenant du poste informatique (1) et notamment :
   - le métamodèle (15), lorsqu'il existe
   - le modèle numérique cible (16)
   - les références aux modèles d'évolution (18) utilisées pour le calcul du modèle numérique d'impression (17)
   - le modèle d'impression numérique (17)
   - les caractéristiques des composants à imprimer
   - les données du cahier de laboratoire électronique sur les conditions de l'impression
- Les données provenant du poste de bioimpression et notamment :
- les images 2D horodatées acquises par la caméra (4) et autres données caractérisant les couches imprimées pendant la phase de bioimpression
- les informations relatives à la séquence de tirs laser, dans le cas d'une bioimpression assistée par laser
- les informations horodatées provenant de capteurs physico-chimiques telles que la température, l'hygrométrie, ... et des capteurs éventuellement présents dans l'objet imprimé.
- Les données provenant du poste de maturation (5) et notamment :
   - les images 3D horodatées acquises par le système d'imagerie (6) pendant la phase de bioimpression et autres caractérisations de l'objet imprimé (biochimiques, spectroscopiques...)
   - les informations horodatées provenant de capteurs physico-chimiques équipant le poste de maturation, telles que la température, l'hygrométrie, etc., et des capteurs éventuellement présents dans l'objet imprimé
   Ces données sont traitées par le calculateur (8) pour identifier les écarts entre l'image 3D transmise par le système d'imagerie (6) pendant la maturation et avec le modèle numérique d'impression (17) à la fin de la maturation. Ce traitement permet de vérifier l'évolution du tissu depuis sa phase de bioimpression, et de calculer les écarts entre l'image 3D transmise par le système d'imagerie (6) et le modèle numérique d'impression (17), écarts qui devront être inférieurs à une valeur seuil.

Ce calcul d'écart est par exemple réalisé par un calcul de distance euclidienne ou par un calcul de distance de Mahalanobis basé sur la détermination d'un indicateur de corrélation (calculé à partir de matrices de covariance) entre le modèle numérique d'impression (17) et l'image 3D transmise par le système d'imagerie (6). Alternativement, le calcul d'écart est réalisé par une méthode de partitionnement de données (« data clustering » en anglais).

Ces données sont également enregistrées dans une mémoire du calculateur (8), pour permettre des traitements d'amélioration des modèles numériques d'évolution (18) enregistrés dans la base de données (9).

Les traitements pour recalculer les modèles d'évolution (18) sont des méthodes d'apprentissage automatique ou statistique (« machine learning » en anglais) connues dans l'état de la technique.

Ces traitements sont appliqués périodiquement sur une collection de données correspondant à un métamodèle (15) commun et résultant d'une pluralité de bioimpressions réalisées.

Si le système comprend un serveur commun à plusieurs plateformes de bioimpression et de maturation (10 à 12), il permet d'augmenter la collecte de données et donc la robustesse de ce traitement de recalcul des modèles d'évolution (18).

Selon une implémentation particulière, le serveur commun est distribué ou dupliqué sur plusieurs serveurs pour bénéficier de la redondance, avec un système de gestion de base de données de type NoSQL.

Dans ce cas, il est avantageux que la bibliothèque de modèles d'évolution (18) soit enregistrée non pas dans une mémoire locale du calculateur (7), mais dans une mémoire commune à toutes les plateformes (10 à 12) connectées à un serveur (8) partagé, qui peut également jouer le rôle de calculateur centralisé ayant de grosses capacités de traitements de données.

### Exemple de modélisation de l'évolution

Selon un exemple non limitatif de réalisation, on enregistre pour chaque type de cellule une représentation numérique de type classe (en langage orienté objet) ayant les fonctions suivantes :
- des attributs comme la position dans un espace de référence (par exemple les coordonnées cartésiennes du centre géométrique de la cellule i à un instant donné, de type xᵢ, yᵢ, zᵢ et t), le type cellulaire, l'âge cellulaire... les données présentes dans le cahier de laboratoire électronique
- des méthodes d'évolution de chaque type cellulaire (faire évoluer, migrer, diviser...) par le biais d'une représentation volumique sous forme d'un nuage de « points » massiques en interaction de deux types :
   - des points internes de N classes de tailles en contact rigide avec amortissement mais sans adhésion ni frottement, avec un modèle d'interaction en compression.
   - Les points membranaires, tous de même taille, sont liés par des éléments de câbles viscoélastiques représentés par un ou plusieurs paramètres (tenseurs) numériques d'interaction avec les autres cellules voisines. Cet ensemble représentant la membrane de la cellule encapsule les points internes avec lesquels ils sont en contact rigide.

Cette modélisation numérique complexe de cellules en 3D dans le temps permet de représenter les mécanismes d'évolution fondamentaux comme la croissance, la quiesence , la différentiation, l'apoptose et encore la division cellulaire.

La croissance d'une cellule peut être modélisée par un recalcul périodique des paramètres numériques de chaque cellule de la manière suivante : tous les n pas de temps, un nouveau point interne est ajouté au centre de la cellule i. Ce nombre entier nᵢ est un paramètre qui définit alors la vitesse de croissance de la cellule (Vcrs_cellule) et qui est déterminé empiriquement en fonction de la dynamique globale du système.
- Si n est trop petit, la vitesse de croissance des cellules est trop grande, les points internes de la cellule n'ont pas le temps de s'organiser, la cellule n'a donc pas une forme stable.
- Si n est trop grand, la cellule croît lentement et la durée de la simulation augmente. En outre, la taille du nouveau point interne ajouté dans la cellule est déterminée en fonction du nombre de points déjà présents. Ainsi, une cellule a un même nombre de points internes de chaque taille à un point près afin de conserver une granulométrie régulière et/ou la forme de la cellule (allongement).

On peut modéliser l'évolution du tissu résultant du développement par accrétion par les paramètres suivants :
- la vitesse de croissance des cellules V_{crs_cellule},
- la vitesse d'arrivée des cellules par division cellulaire V_{accrétion},
- la vitesse de croissance de la sphère V_{crs_sphère}.

L'ensemble de ces paramètres définit une vitesse de développement du tissu.

Le calcul dynamique d'évolution temporelle d'un tissu est réalisé par un programme informatique prenant en compte l'évolution temporelle de chaque cellule et les interactivités intracellulaires voisines.

Les étapes du développement modélisées par des paramètres numériques sont les suivantes :
- détermination des cellules entrant en division,
- début de la division : création des éléments d'actine,
- évolution de la division : réduction des éléments d'actine,
- fin de la division : la cellule mère donne deux cellules filles,
- évolution des éléments de type « cadhérine » qui influent directement sur l'adhésion cellulaire,
- détermination des cellules devant entrer en apoptose,
- évolution de l'apoptose : suppression de points et/ou de cellule.

A chaque passage dans la boucle « évolution du tissu », toutes les opérations ne sont pas prises en compte pour chaque cellule et seules les méthodes adaptées s'exécutent. Par exemple, une cellule qui n'est pas différenciée ne peut pas rentrer en quiesence et une cellule en division ne peut pas entrer en apoptose.

L'évolution temporelle de chaque cellule prend en compte des conditions aux limites et des hypothèses d'évolution dépendant du temps. Ce calcul est réalisé de manière itérative pour déterminer à chaque pas les nouvelles positions et vitesses pour chaque point. L'évolution structurelle du système, c'est à dire l'ajout ou la suppression de points ou d'éléments, en fonction des données issues des calculs mécaniques et en suivant les lois d'évolution qui régissent le modèle est réalisée en fonction du modèle d'évolution de la division et de l'apoptose.

Les cellules et le tissu sont construits en fonction des données enregistrées dans un fichier définissant la configuration initiale (positionnement des points, caractéristiques des points et des éléments, etc.) et les paramètres de la morphogenèse numérique : type de scénario (tissu formé par accrétion ou par prolifération), de division, etc. Ensuite, régulièrement au cours de la simulation, les membranes de chaque cellule sont mises à jour (allongement, diminution, suppression et ajout de câbles membranaires conformément aux règles d'évolution de la membrane définies au paragraphe 3.2.1), et ce, quel que soit le type de scénario. Puis, en fonction du choix de développement de tissu initialisé, l'algorithme de « tissu non prolifératif » ou « tissu prolifératif » s'exécute. Les cellules sont ainsi modifiées suivant les lois précédemment définies (type de division, d'apoptose, de croissance, etc.). De la même manière, la modélisation prend en compte l'évolution de la matrice extracellulaire (densité, rigidité, signalisation moléculaire, etc.).

Le pas de temps choisi est par exemple de 0,5 secondes pour recalculer l'évolution du tissu entre deux itérations.

A titre d'exemple, les paramètres numériques et les fonctions d'évolution sont les suivants :

| | |
|---|---|
| vitesse de croissance de la cellule : Vcrs_cellule | ajout d'un nœud tous les 100 pas de temps |
| vitesse d'accrétion des cellules : Vaccrétion | ajout de cellule tous les 50 pas de temps |
| vitesse de croissance de la sphère : Vcrs_sphère | augmentation de 5% de la surface de la sphère tous les 50 pas de temps |
| vitesse d'apoptose : Vapop | suppression de 5 points tous les 100 pas de temps |
| renouvellement des câbles « cadhérines » | mise à jour tous les n_cad pas de temps : n_cad = 50 |
| distance minimale pour créer un élément cadhérine | distance minimale D_cad entre deux points : D_cad = 13 m |

Cette modélisation permet de déterminer un modèle numérique intermédiaire (32) dont l'évolution conduira à un tissu ou produit cible (30) correspondant à un fichier numérique (40).

La détermination peut se faire par test de différents modèles numériques intermédiaires (32) et d'estimation des fichiers numériques (40) cibles correspondant, pour sélectionner le fichier numérique (40) se rapprochant le plus du tissu attendu.

### Présentation fonctionnelle

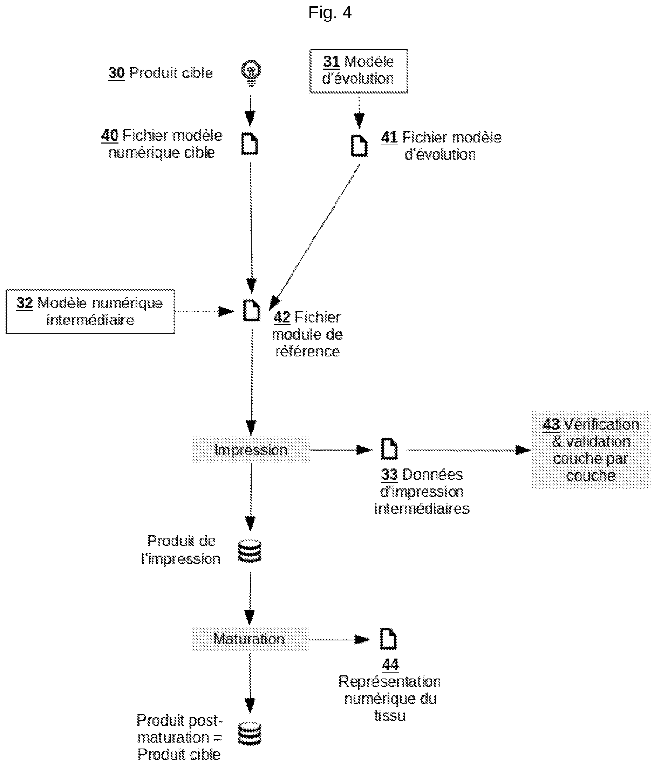

La figure 4 illustre schématiquement le principe mis en œuvre par l'invention.

La première étape consiste à définir la structure tridimensionnelle du tissu que l'on souhaite réaliser, sous forme d'un cahier de laboratoire électronique ou d'un fichier de CAO. Ce fichier définit la structure visée avec l'implantation théorique des cellules et particules dans un réseau tridimensionnel se traduisant par un fichier numérique (40) qui spécifie le produit cible (30). La caractérisation de la structure 3D peut être mutli-modale, multi-échelle et automatisée.

Par ailleurs, un modèle d'évolution (41) décrivant les évolutions des différentes cellules et particules conduit à un fichier ou une série de fichiers numériques définissant le modèle d'évolution (31). Le modèle d'évolution prend en compte des évolutions endogènes, telles que le vieillissement, l'évolution de la taille, la différentiation, la disparition ou le dédoublement, en fonction du temps et d'autres paramètres d'environnement tels que la température, ou l'évolution de la composition physico-chimique du substrat, pour une particule ou cellule isolée. Il prend aussi en compte des paramètres exogènes tels que la présence, la nature et la distance d'autres particules ou cellules qui exercent des forces mécaniques et émettent des signaux paracrines.

Ces deux séries de fichiers (40 et 41) font l'objet d'un traitement pour calculer un module de référence (42) par application d'un modèle numérique intermédiaire (32), consistant à déterminer une structure tridimensionnelle initiale dont l'évolution, en application du modèle d'évolution (41), donne un résultat conforme au produit cible (30), par une fonction d'estimation inverse ou de manière empirique, par détermination d'une hypothèse de modèle de référence, d'application du modèle d'évolution et de comparaison du résultat avec le produit cible (30), de manière récursive pour minimiser les écarts entre le résultat de l'application du modèle d'évolution sur le modèle de référence (42) et le produit cible (30).

Ce modèle de référence (42) pilotera le processus de bioimpression qui produira un tissu dont la construction est caractérisée couche par couche pour fournir des données d'impression intermédiaire (33) soumises à des vérifications (43) et des étapes de validation ou de correction.

Selon une variante l'objet est mis ensuite en maturation. Son évolution est également analysée par des moyens de mesure et de contrôle, notamment d'imagerie pour fournir une représentation numérique (44) du tissu à différents stades d'évolution.

Selon d'autres variantes, l'objet est implanté directement ou très rapidement après sa fabrication, on parle alors de maturation partielle.

Selon une autre variante ultime, l'objet est directement imprimé in situ, notamment in vivo sur l'animal ou le patient. Il n'y a pas dans ce cas de figure de maturation. Elle se fait directement sur le lieu d'implantation.

## Revendications

1. Procédé de bioimpression comportant :
• une étape de préparation d'un modèle numérique cible (16) représentatif de l'organisation tridimensionnelle du tissu à fabriquer,
• une étape de pilotage d'un équipement de bioimpression (2) pour le dépôt d'une pluralité de couches de cellules vivantes et de biomatériaux
**caractérisé en ce qu'**il comporte en outre
• une étape de calcul d'un modèle numérique d'impression (17) en fonction dudit modèle numérique du produit à fabriquer (16), d'une part, et d'un modèle prédictif d'évolution (18) d'autre part, ainsi que des caractéristiques des constituants à imprimer
• l'étape de pilotage de l'équipement de bioimpression (2) étant réalisée selon ledit modèle numérique d'impression (17) ainsi calculé.

2. Procédé de bioimpression selon la revendication 1 **caractérisé en ce qu'**il comporte en outre une étape de caractérisation 2D de chacune des couches pendant l'étape de bioimpression.

3. Procédé de bioimpression selon la revendication 1 **caractérisé en ce qu'**il comporte en outre une étape de maturation de l'objet imprimé.

4. Procédé de bioimpression selon la revendication 1 **caractérisé en ce qu'**il comporte en outre une étape de caractérisation 3D du produit bioimprimé juste après la bioimpression.

5. Procédé de bioimpression selon la revendication 1 **caractérisé en ce qu'**il comporte en outre une étape de caractérisation 3D du produit bioimprimé au cours de sa maturation.

6. Procédé de bioimpression selon la revendication 1 **caractérisé en ce que** ledit modèle prédictif d'évolution (18) est enregistré sur un serveur partagé (8), lesdits équipements comportant des moyens de communication avec ledit serveur pour sélectionner dans une bibliothèque numérique (9) un modèle prédictif d'évolution (18) enregistré adapté à une catégorie de produits cibles.

7. Procédé de bioimpression selon la revendication 1 **caractérisé en ce qu'**il comporte des étapes de traitement de type multi-agent sur les données provenant d'au moins un équipement connecté (10 à 12) pour le recalcul périodique des modèles prédictif d'évolution (17).

8. Procédé de bioimpression selon la revendication 1 **caractérisé en ce qu'**il comporte des étapes de traitement de type apprentissage par machine sur les données provenant d'au moins un équipement connecté (10 à 12) pour le recalcul périodique des modèles prédictif d'évolution (17).

9. Procédé de bioimpression selon la revendication 1 **caractérisé en ce qu'**il comporte des étapes de traitement de type modélisation continue par équations différentielles sur les données provenant d'au moins un équipement connecté (10 à 12) pour le recalcul périodique des modèles prédictif d'évolution (17).

10. Procédé de bioimpression selon la revendication 6 **caractérisé en ce que** les équipements connectés (10 à 12) transmettent audit serveur connecté des informations numériques horodatées, descriptives du tissu cible, de l'environnement de celui-ci, et des évènements détectés pendant l'étape de bioimpression et l'étape de maturation.

11. Procédé de bioimpression selon la revendication 1 **caractérisé en ce qu'**il comporte une étape de calcul d'une pluralité de modèles d'impression et des étapes d'évaluation des évolutions théoriques des modèles d'impression ainsi calculés, et de sélection du modèle d'impression (17) minimisant les écarts entre le modèle cible et le modèle maturé calculé.

12. Procédé de bioimpression selon la revendication 1 **caractérisé en ce que** l'étape de calcul du modèle d'impression (17) met en œuvre un traitement de convolution entre le modèle cible (16) et un modèle prédictif d'évolution (18).

13. Système de bioimpression comportant :
- un poste de bioimpression équipé
∘ d'au moins une tête de bioimpression (2) apte à commander le transfert de biomatériaux et/ou de cellules vivantes vers une cible,
∘ et de moyens de caractérisation 2D (4) pour l'enregistrement de données de chacune des couches pendant l'étape de bioimpression
- ainsi qu'un calculateur (7) commandant ledit poste de bioimpression, comportant des moyens de définition et d'enregistrement dans la mémoire du calculateur d'un modèle numérique cible représentatif de l'organisation tridimensionnelle du produit à fabriquer,
**caractérisé en ce qu'**il comporte un calculateur (8) associé à une mémoire (9) pour l'enregistrement d'une pluralité de modèles numériques prédictif d'évolution (17) et exécute un programme commandant :
- une étape de calcul d'un modèle numérique d'impression (17) calculé en fonction
∘ dudit modèle numérique cible (16) du produit à fabriquer, d'une part, et
∘ d'un modèle prédictif d'évolution (17) d'autre part, et
∘ des caractéristiques des constituants à imprimer, et
- une étape de pilotage dudit poste de bioimpression (2) en application dudit modèle numérique d'impression ainsi calculé.

14. Système de bioimpression selon la revendication 13 **caractérisé en ce qu'**il comporte au moins un poste de maturation (5) des cibles réalisées par bio-impression.

15. Système de bioimpression selon la revendication 13 **caractérisé en ce qu'**il comporte une pluralité de postes de bioimpression (2), chacun desdits postes de bioimpression (2) comportant des moyens de communication avec un serveur commun (8) comportant ledit calculateur, ledit calculateur transmettant à chaque calculateur local (7) associé à un poste de bioimpression (2) le modèle numérique (16) à appliquer par la tête de bioimpression (2) en fonction de chaque application visée.

## Patentansprüche

1. Biodruckverfahren, das Folgendes umfasst:
• einen Schritt der Vorbereitung eines digitalen Zielmodells (16), das die dreidimensionale Organisation des herzustellenden Gewebes darstellt,
• einen Schritt der Steuerung einer Biodruckeinrichtung (2) für die Abscheidung mehrerer Schichten lebender Zellen und Biomaterialien,
**dadurch gekennzeichnet, dass** es ferner Folgendes umfasst
• einen Schritt der Berechnung eines digitalen Druckmodells (17) in Abhängigkeit von dem digitalen Modell des herzustellenden Produkts (16) einerseits und einem Entwicklungsvorhersagemodell (18) andererseits, sowie Eigenschaften der zu druckenden Bestandteile,
• wobei der Schritt der Steuerung der Biodruckeinrichtung (2) gemäß dem so berechneten digitalen Druckmodell (17) ausgeführt wird.

2. Biodruckverfahren nach Anspruch 1, **dadurch gekennzeichnet, dass** es ferner während des Biodruckschritts einen Schritt einer 2D-Charakterisierung von jeder der Schichten umfasst.

3. Biodruckverfahren nach Anspruch 1, **dadurch gekennzeichnet, dass** es ferner einen Schritt der Reifung des gedruckten Objekts umfasst.

4. Biodruckverfahren nach Anspruch 1, **dadurch gekennzeichnet, dass** es ferner einen Schritt einer 3D-Charakterisierung des biogedruckten Produkts kurz nach dem Biodrucken umfasst.

5. Biodruckverfahren nach Anspruch 1, **dadurch gekennzeichnet, dass** es ferner einen Schritt der 3D-Charakterisierung des biogedruckten Produkts im Lauf seiner Reifung umfasst.

6. Biodruckverfahren nach Anspruch 1, **dadurch gekennzeichnet, dass** das Entwicklungsvorhersagemodell (18) auf einem gemeinsam genutzten Server (8) aufgezeichnet wird, wobei die Einrichtungen Mittel zum Kommunizieren mit dem Server zum Auswählen eines aufgezeichneten Entwicklungsvorhersagemodells (18) in einer digitalen Bibliothek (9) umfassen, das für eine Kategorie von Zielprodukten geeignet ist.

7. Biodruckverfahren nach Anspruch 1, **dadurch gekennzeichnet, dass** es Schritte der Verarbeitung der Multiagentenart an den Daten, die von wenigstens einer angeschlossenen Einrichtung (10 bis 12) stammen, zur periodischen Neuberechnung der Entwicklungsvorhersagemodelle (17) umfasst.

8. Biodruckverfahren nach Anspruch 1, **dadurch gekennzeichnet, dass** es Schritte der Verarbeitung einer Maschinenlernart an den Daten, die von wenigstens einer angeschlossenen Einrichtung (10 bis 12) stammen, zur periodischen Neuberechnung der Entwicklungsvorhersagemodelle (17) umfasst.

9. Biodruckverfahren nach Anspruch 1, **dadurch gekennzeichnet, dass** es Schritte der Verarbeitung einer Art der fortlaufenden Modellierung durch Differentialgleichungen an den Daten, die von wenigstens einer angeschlossenen Einrichtung (10 bis 12) stammen, zur periodischen Neuberechnung der Entwicklungsvorhersagemodelle (17) umfasst.

10. Biodruckverfahren nach Anspruch 6, **dadurch gekennzeichnet, dass** die angeschlossenen Einrichtungen (10 bis 12) mit Zeitstempel versehene digitale Informationen an den angeschlossenen Server übermitteln, die das Zielgewebe, seine Umgebung und die während des Schrittes des Biodruckens und des Schrittes der Reifung erfassten Ereignisse beschreiben.

11. Biodruckverfahren nach Anspruch 1, **dadurch gekennzeichnet, dass** es einen Schritt der Berechnung mehrerer Druckmodelle und Schritte der Bewertung der theoretischen Entwicklungen der so berechneten Druckmodelle und der Auswahl des Druckmodells (17), das die Abweichungen zwischen dem Zielmodell und dem berechneten gereiften Modell minimiert, umfasst.

12. Biodruckverfahren nach Anspruch 1, **dadurch gekennzeichnet, dass** der Schritt der Berechnung des Druckmodells (17) eine Konvolutionsverarbeitung zwischen dem Zielmodell (16) und einem Entwicklungsvorhersagemodell (18) implementiert.

13. Biodrucksystem, das Folgendes umfasst:
- eine eingerichtete Biodruckstation
∘ wenigstens einen Biodruckkopf (2), der fähig ist, die Übertragung von Biomaterialien und/oder lebenden Zellen in Richtung eines Ziels zu steuern,
∘ und 2D-Charakterisierungsmittel (4) zur Aufzeichnung von Daten von jeder der Schichten während des Schrittes des Biodruckens
- sowie eine Recheneinrichtung (7), die die Biodruckstation steuert, die Mittel zur Definition und zur Aufzeichnung eines digitalen Zielmodells, das die dreidimensionale Organisation des herzustellenden Produkts darstellt, in dem Speicher der Recheneinrichtung umfasst,
**dadurch gekennzeichnet, dass** es eine Recheneinrichtung (8) umfasst, die mit einem Speicher (9) zur Aufzeichnung mehrerer digitaler Entwicklungsvorhersagemodelle (17) verbunden ist und ein Programm ausführt, das Folgendes steuert:
- einen Schritt der Berechnung eines digitalen Druckmodells (17), das in Abhängigkeit von Folgendem berechnet wird:
∘ dem digitalen Zielmodell (16) des herzustellenden Produkts einerseits und
∘ einem Entwicklungsvorhersagemodell (17) andererseits und
∘ den Eigenschaften der zu druckenden Bauteile und
- einen Schritt des Steuerns der Biodruckstation (2) bei Anwendung des so berechneten digitalen Druckmodells.

14. Biodrucksystem nach Anspruch 13, **dadurch gekennzeichnet, dass** es wenigstens eine Reifungsstation (5)von durch Biodrucken ausgeführten Zielen umfasst.

15. Biodrucksystem nach Anspruch 13, **dadurch gekennzeichnet, dass** es mehrere Biodruckstationen (2) umfasst, wobei jede der Biodruckstationen (2) Mittel zur Kommunikation mit einem gemeinsamen Server (8) umfasst, der die Recheneinrichtung umfasst, wobei die Recheneinrichtung an jede lokale Recheneinrichtung (7), die einer Biodruckstation (2) zugeordnet ist, das digitale Modell (16) übermittelt, das durch den Biodruckkopf (2) in Abhängigkeit von jeder angestrebten Anwendung anzuwenden ist.

## Claims

1. Bioprinting method, comprising:
• a step of preparing a target digital model (16) that represents the three-dimensional organization of the tissue to be manufactured,
• a step of controlling a bioprinting device (2) to deposit a plurality of layers of living cells and biomaterials,
**characterized in that** it further comprises
• a step of calculating a digital printing model (17) on the basis of said digital model (16) of the product to be manufactured, a predictive evolution model (18), and the properties of the components to be printed,
• the step of controlling the bioprinting device (2) being carried out according to said thus calculated digital printing model (17).

2. Bioprinting method according to claim 1, **characterized in that** it further comprises a step of 2D characterization of each of the layers during the bioprinting step.

3. Bioprinting method according to claim 1, **characterized in that** it further comprises a step of maturing the printed object.

4. Bioprinting method according to claim 1, **characterized in that** it further comprises a step of 3D characterization of the bioprinted product immediately after the bioprinting.

5. Bioprinting method according to claim 1, **characterized in that** it further comprises a step of 3D characterization of the bioprinted product during the maturation thereof.

6. Bioprinting method according to claim 1, **characterized in that** said predictive evolution model (18) is stored on a shared server (8), said devices comprising means of communication with said server for selecting, from a digital library (9), a stored predictive evolution model (18) adapted to a category of target products.

7. Bioprinting method according to claim 1, **characterized in that** it comprises processing steps of the multi-agent-type on the data from at least one connected device (10 to 12) for the periodic recalculation of the predictive evolution models (17).

8. Bioprinting method according to claim 1, **characterized in that** it comprises processing steps of the machine learning-type on the data from at least one connected device (10 to 12) for the periodic recalculation of the predictive evolution models (17).

9. Bioprinting method according to claim 1, **characterized in that** it comprises processing steps of the continuous-modeling-type using differential equations on the data from at least one connected device (10 to 12) for the periodic recalculation of the predictive evolution models (17).

10. Bioprinting method according to claim 6, **characterized in that** the connected devices (10 to 12) transmit, to said connected server, time-stamped digital information describing the target tissue, the environment thereof, and the events detected during the bioprinting step and the maturing step.

11. Bioprinting method according to claim 1, **characterized in that** it comprises a step of calculating a plurality of printing models and steps of evaluating the theoretical evolutions of the thus calculated printing models and of selecting the printing model (17) that minimizes the differences between the target model and the calculated mature model.

12. Bioprinting method according to claim 1, **characterized in that** the step of calculating the printing model (17) implements convolution between the target model (16) and a predictive evolution model (18).

13. Bioprinting system, comprising:
- a bioprinting station provided with
∘ at least one bioprinting head (2) capable of controlling the transfer of biomaterials and/or living cells to a target,
∘ 2D characterization means (4) for storing data of each of the layers during the bioprinting step,
- and a computer (7) that controls said bioprinting station and comprises means for defining and storing, in the memory of the computer, a target digital model that represents the three-dimensional organization of the product to be manufactured,
**characterized in that** it comprises a computer (8) which is associated with a memory (9) for storing a plurality of predictive digital evolution models (17) and runs a program that controls:
- a step of calculating a digital printing model (17) calculated on the basis of
∘ said target digital model (16) of the product to be manufactured and
∘ a predictive evolution model (17), and
∘ the properties of the components to be printed, and
- a step of controlling said bioprinting station (2) according to said thus calculated digital printing model.

14. Bioprinting system according to claim 13, **characterized in that** it comprises at least one maturing station (5) for the targets produced by bioprinting.

15. Bioprinting system according to claim 13, **characterized in that** it comprises a plurality of bioprinting stations (2), each of said bioprinting stations (2) comprising means of communication with a common server (8) comprising said computer, said computer transmitting, to each local computer (7) associated with a bioprinting station (2), the digital model (16) to be applied by the bioprinting head (2) according to each intended application.
